# EUROPÄISCHE PATENTSCHRIFT

(11) **EP 0 378 073 B1**
(45) Veröffentlichungstag und Bekanntmachung des Hinweises auf die Patenterteilung: **19.05.1993**
(21) Anmeldenummer: 90100030.7
(22) Anmeldetag: 02.01.1990
(51) Int. Cl.: C07C 45/51, C07C 49/395, C07C 69/734

(54) **Verfahren zur Herstellung von 1,3-Cyclopentandion**
Process for the preparation of 1,3-cyclopentandione
Procédé pour la préparation de 1,3-cyclopentanedione

(30) Priorität: 06.01.1989 CH 41/89
(43) Veröffentlichungstag der Anmeldung: 18.07.1990
(73) Patentinhaber: LONZA AG, CH-4002 Basel (CH)
(72) Erfinder: Fuchs, Rudolf, Dr., SION (Kanton Wallis) (CH); McGarrity, John, Dr., VISP (Kanton Wallis) (CH)
(74) Vertreter: Weinhold, Peter, Dr.

(56) Entgegenhaltungen:
- DE-A- 2 458 209

## Beschreibung

1,3-Cyclopentandion bzw. seine Derivate sind interessante Zwischenprodukte für zahlreiche Wirkstoffsynthesen, z.B. gemäss CA 107:197620 als Ausgangsprodukt für die Herstellung eines Prostaglandinzwischenprodukts oder gemäss Tetrahedron Letters Vol.22, No.44, S.4385ff. (1981) als Basissubstanzen für das Antibiotikum Kjellmanianon.

Weitere Anwendungen sind in Aldrichimica Acta Vol.10, No.1, (1977) S.19 aufgeführt.

Ebenso zahlreich sind die bekannten Verfahren zur Herstellung von 1,3-Cyclopentandion.

Nach weitverbreiteten Synthesemethoden wird 1,3-Cyclopentadien als Ausgangsprodukt eingesetzt, das darauffolgend in das 1,3-Cyclopentendiol, durch Oxidation in das 1,3-Cyclopentendion und nach abschliessender Hydrierung in das 1,3-Cyclopentandion umgewandelt wird.

Wie von Lick et al. in Chem.Ber.111 (1978) S.2466 dargestellt wird, sind diese Methoden mit zahlreichen Schwierigkeiten verbunden und zur Herstellung grösserer Mengen wenig erfolgreich. Dieselben Autoren haben ein eigenes Verfahren entwickelt, bei dem 2-Norbornen in einer dreistufigen Synthese mit einer Ausbeute von 70% in das 1,3-Cyclopentandion übergeführt wird (Chem. Ber. 111 (1978) S.2461f.).

Durch die verfahrensbedingte zweimal notwendige Ozonspaltung bei -60 bis -70°C ist das Verfahren aufgrund des Energieaufwands (Kühlung, Ozonerzeugung) für die Umsetzung in ein technisches Verfahren wenig rentabel.

Es bestand daher die Aufgabe, ein Verfahren zur Herstellung von 1,3-Cyclopentandion zu entwickeln, das die genannten Nachteile nicht aufweist.

Es wurde gefunden, dass mit einem Verfahren nach Patentanspruch 1 die Aufgabe auf überraschend gute Weise gelöst werden konnte.

Massgebend für die Wirtschaftlichkeit des Verfahrens ist, dass von grosstechnisch erhältlichen Malonsäureestern und von aus Diketen bzw. 4-Halogenacetessigsäureestern leicht erhältlichen 4-Halogen-3-alkoxy-2E-butensäurealkylestern ausgegangen werden kann.

### Stufe a):

Gemäss Patentanspruch 1 wird in der ersten Stufe ein Malonsäureester in Gegenwart einer Base mit einem 4-Halogen-3-alkoxy-2E-butensäureester zum entsprechenden 5,5-Bis-(alkoxycarbonyl)-3-alkoxy-2E-pentensäureester umgesetzt. Zweckmässig werden die Malonsäuredi-(C₁-C₄)-alkylester, vorzugsweise die Malonsäuredi-(C₁-C₂)-alkylester, eingesetzt.

Als geeignete Derivate der 4-Halogen-3-alkoxy-2E-butensäureester sind die 4-Halogen-3-alkoxy-2E-butensäure-(C₁-C₄)-alkylester, als besonders geeignet die 4-Chlor-3-(C₁-C₂)-alkoxy-2E-butensäure-(C₁-C₂)-alkylester, anzusehen.

Als Base können Alkalialkoholate, Alkalihydroxide oder starke organische Basen, vorzugsweise Alkalialkoholate, eingesetzt werden. Mit dem Begriff Alkalialkoholat werden die Natrium- oder Kaliumalkoholate der niederen aliphatischen, gegebenenfalls verzweigten, Alkohole wie z.B. Methanol, Ethanol, Pro panol oder Butanol verstanden.

Geeignete Vertreter der Alkalihydroxide sind Kaliumhydroxid oder Natriumhydroxid und als starke organische Base kann z.B. DBU (1,8-Diazabicyclo(5,4,0)undec-7-en) eingesetzt werden. Die Umsetzung verläuft zweckmässig in Gegenwart eines polaren Lösungsmittels aus der Reihe Acetonitril, Benzonitril, niedere aliphatische Alkohole wie z.B. Methanol oder Ethanol, 1,2-Dimethoxyethan, N,N'-Dimethylformamid oder N,N'-Dimethylacetamid. Bevorzugtes Lösungsmittel ist N,N'-Dimethylformamid.

Die Umsetzungstemperatur wird vorteilhaft zwischen 0 und 180°C, besonders bevorzugt zwischen 20 und 60°C gewählt.

Als Reaktionsprodukt erhält man die bisher nicht bekannten 5,5-Bis-(alkoxycarbonyl)-3-alkoxy-2E-pentensäureester der allgemeinen Formel

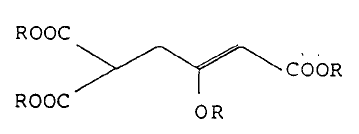

Den Ester- bzw. Alkoxygruppen der eingesetzten Malonsäureester bzw. der 4-Halogen-3-alkoxy-2E-butensäureester entsprechend sind die Reste R gleich oder verschieden und bedeuten Alkyl mit 1 bis 4 C-Atomen.

Für die Synthese von 1,3-Cyclopentandion besonders vorteilhaft sind die 5,5-Bis-((C₁-C₂)-alkoxycarbonyl)-3-(C₁-C₂)-alkoxy-2E-pentensäure-(C₁-C₂)-alkylester.

Die genannten Verbindungen können auf übliche Weise isoliert werden, in der Regel werden sie aber ohne spezielle Aufbereitung direkt für die Folgestufe b) eingesetzt.

### Stufe b):

Die Stufe b) beinhaltet den Ringschluss der 5,5-Bis-(alkoxycarbonyl)-3-alkoxy-2E-pentensäureester in Gegenwart einer Base.

Als Basen werden der Stufe a) entsprechend entweder die Natrium- oder Kaliumalkoholate der niederen aliphatischen Alkohole Methanol, Ethanol, Propanol oder Butanol oder die Alkalihydroxide Kaliumhydroxid oder Natriumhydroxid eingesetzt.

Lösungsmittel für den Ringschluss ist in der Regel der dem Alkoholat entsprechende Alkohol. Es ist aber durchaus möglich, andere polare Lösungsmittel wie z.B. Acetonitril oder Benzonitril zu verwenden.

Der Ringschluss erfolgt zweckmässig bei Temperaturen zwischen 0 und 180°C, vorzugsweise zwischen 20 und 60°C.

Als Reaktionsprodukt wird das entsprechende Salz des 5-Alkoxycarbonyl-3-alkoxy-2-cyclopenten-1-ons der Formel 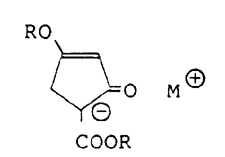
worin M Natrium oder Kalium bedeutet und R die genannte Bedeutung hat, erhalten.

### Stufen c) und d):

Zur Gewinnung des 1,3-Cyclopentandions wird die Estergruppe des Salzes aus Stufe b) verseift (Stufe c) und die Carboxylgruppe schliesslich decarboxyliert.

Zweckmässige Basen für die Verseifung sind wässrige Lösungen von Natrium- oder Kaliumhydroxid.

Die Verseifungstemperatur liegt vorteilhaft zwischen 0 und 100°C, bevorzugt ist Raumtemperatur.

Das resultierende Carbonsäuresalz wird in der Regel nicht isoliert, sondern in situ mit einer Säure weiter behandelt. Geeignete Säuren sind die anorganischen Mineralsäuren, wie z.B. Salzsäure oder Schwefelsäure.

Mit der Säurebehandlung, die zweckmässig bei Temperaturen zwischen 20 und 100°C erfolgt, geht die Decarboxylierung und damit Ueberführung in das 1,3-Cyclopentandion einher.

Nach üblicher Aufarbeitung kann das gewünschte Produkt in guter Qualität und guten Ausbeuten erhalten werden.

Die folgenden Beispiele sollen das erfindungsgemässe Verfahren näher erläutern.

### Beispiele

### Beispiel 1

### a) Herstellung von 5,5-Bis-(ethoxycarbonyl)-3-methoxy-2E-pentensäuremethylester

83,9 g (0,5 Mol) Malonsäurediethylester wurden in 250 ml N,N'-Dimethylformamid vorgelegt. Bei 20°C gab man 27,8 g (0,5 Mol) Natriummethylat zu, 10 Minuten später innerhalb von 5 Minuten gab man 41,6 g (0,25 Mol) 4-Chlor-3-methoxy-2E-butensäuremethylester zu. Bei 20°C wurde während 2 Stunden gerührt. Danach gab man nochmals 10,8 g (0,2 Mol) Natriummethylat zu. Nach 15stündigem Rühren bei 20°C wurde N,N'-Dimethylformamid bei 40 bis 50°C/20 mbar abdestilliert. Dem Rückstand wurden 120 ml Wasser und 100 ml Methylenchlorid zugegeben. Nach Neutralisation der Phasen wurde die organische Phase abgetrennt und zur Trockene eingedampft. Der Rückstand wurde im Vakuum bei 176 bis 180°C/20 mbar destilliert. Man erhielt 58,8 g (80%) des Titelprodukts, mit einer Reinheit von 98% (GC).
¹H-NMR: (CDCl₃, 300 MHz) δ
1,25, t J = 6 Hz, 6H
3,4, d J = 8 Hz, 2H
3,6, s, 3H
3,68, s, 3H
3,71, t J = 8 Hz, 1H
4,16, q J = 6 Hz, 4H
5,06, s, 1H

### a1) Herstellung von 5,5-Bis-(methoxycarbonyl)-3-methoxy-2E-pentensäuremethylester

66 g (0,5 Mol) Malonsäuredimethylester wurden in 250 ml N,N'-Dimethylformamid vorgelegt. Bei 20°C gab man 27,8 g (0,5 Mol) Natriummethylat zu, 10 Minuten später innerhalb von 5 Minuten gab man 41,6g (0,25 Mol) 4-Chlor-3-methoxy-2E-pentensäuremethylester zu. Bei 20°C wurde während 2 Stunden gerührt. Danach gab man nochmals 10,8 g (0,2 Mol) Natriummethylat zu. Nach 15stündigem Rühren bei 20°C wurde das Lösungsmittel abdestilliert. Nach Aufarbeitung entsprechend Beispiel a) wurden 53,8 g (81%) des Titelproduktes, mit einer Reinheit von 98% (GC) erhalten.
¹H-NMR: (CDCl₃, 300 MHz) δ
3,4, d, J = 8 Hz, 2H
3,60, s, 3H
3,66, s, 3H
3,71, s, 6H
3,7, t, J = 8 Hz, 1H
5,1, s, 1H

### a2) Herstellung von 5,5-Bis-(ethoxycarbonyl)-3-ethoxy-2E-pentensäureethylester

83,9 g (0,5 Mol) Malonsäurediethylester wurden in 250 ml N,N'-Dimethylformamid vorgelegt. Bei 20°C gab man 27,8 g (0,5 Mol) Natriummethylat zu, 10 Minuten später innerhalb von 5 Minuten gab man 48 g (0,25 Mol) 4-Chlor-3-ethoxy-2E-pentensäureethylester zu. Bei 20°C wurde während 2 Stunden gerührt. Danach gab man nochmals 10,8 g (0,2 Mol) Natriummethylat zu. Nach 15stündigem Rühren bei 20°C wurde das Lösungsmittel abdestilliert. Nach Aufarbeitung entsprechend Beispiel a) wurden 63,75 g (79%) des Titelproduktes, mit einer Reinheit von 98% (GC) erhalten.
¹H-NMR: (CDCl₃, 300 MHz) δ
1,2-1,35, m, 12H
3,41, d, J = 8 Hz, 2H
3,71, t, J = 8Hz, 1H
3,80, q, J = 8Hz, 2H
4,1-4,18, m, 6H
5,02, s, 1H

### b) Herstellung von 5-Ethoxycarbonyl-3-methoxy-2-cyclopenten-1-on-Na-salz

9 g (56 mMol) Malonsäurediethylester wurden in 50 ml N,N'-Dimethylformamid vorgelegt. Bei 20°C gab man 3 g (56 mMol) Natriummethylat zu, 10 Minuten später innerhalb von 5 Minuten gab man 8,25 g (50 mMol) 4-Chlor-3-methoxy-2E-butensäuremethylester zu. Bei 20°C rührte man 1,5 Stunden, nach der zusätzlichen Zugabe von 0,9 g (17 mMol) Natriummethylat rührte man nochmals 1,5 Stunden. Das N,N'-Dimethylformamid wurde darauf bei 40 bis 50°C/20 mbar abdestilliert. Dem Rückstand wurden 100 ml Wasser und 100 ml Methylenchlorid zugegeben. Die organische Phase wurde abgetrennt und die Wasserphase wurde mit 80 ml Methylenchlorid extrahiert. Die vereinigten organischen Phasen wurden eingedampft und der Rückstand in 100 ml Ethanol gelöst. Dieser Lösung wurden 3,5 g (51 mMol) Natriumethylat, gelöst in 100 ml Ethanol während 15 Minuten bei 20°C zugeben. Nach 2 Stunden Rühren bei 60°C fiel das Titelprodukt aus. Bei 60°C wurden darauf 100 ml Toluol zugetropft und auf 0°C abgekühlt. Nach Filtration und Trocknung erhielt man 5,43 g (52,5%) des Titelproduktes.
¹H-NMR: (CDCl₃, 300 MHz) δ (freies Produkt)
1,30, t, J = 7,5 Hz, 3H
2,79, dd, J1 = 18 Hz, J2 = 7,5 Hz, 1H
3,06, dd, J1 = 18 Hz, J3 = 3 Hz, 1H
3,52, dd, J2 = 7,5 Hz, J3 = 3 Hz, 1H
3,89, s, 3H
4,24, q, J = 7,5 Hz, 2H
5,30, s, 1H

### c) Herstellung von 1,3-Cyclopentandion

2,1 g (10 mMol) 5-Ethoxycarbonyl-3-methoxy-2-cyclopenten-1-on-Na-salz wurden in 20 ml Wasser und 5 ml Natronlauge (4 N) vorgelegt. Man rührte 2,5 Stunden bei 20°C. Danach wurden 2,6 g Salzsäure (32% in H₂O) zugegeben (pH 3). Die Lösung wurde 2 Stunden bei 90°C gerührt und dann im Vakuum eingedampft. Der Rückstand wurde in 10 ml Ethanol aufgeschlämmt und filtriert.

Die Ethanolphase wurde eingeengt. Es wurden 0,85 g (80%) 1,3-Cyclopentandion erhalten. Fp. 145 bis 147°C.
¹H-NMR: (DMSO, 300 MHz) δ
2,38, s, 4H
5,10, s, 1H
11,5-12,5, br. s, 1H

## Patentansprüche

1. Verfahren zur Herstellung von 1,3-Cyclopentandion dadurch gekennzeichnet, dass man in einer ersten Stufe a) einen Malonsäureester in Gegenwart einer Base mit einem 4-Halogen-3-alkoxy-2E-butensäureester in einen 5,5-Bis-(alkoxycarbonyl)-3-alkoxy-2E-pentensäureester der Formel 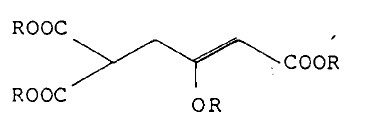 worin die Reste R jeweils gleich oder verschieden sind und Alkyl mit 1 bis 4 C-Atomen bedeuten, umsetzt, diesen in der zweiten Stufe b) in Gegenwart einer Base zum entsprechenden Salz des 5-Alkoxycarbonyl-3-alkoxy-2-cyclopenten-1-ons der Formel 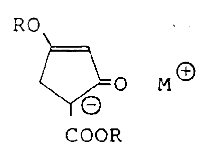 worin M Natrium oder Kalium bedeutet und R die genannte Bedeutung hat, ringschliesst, in Stufe c) in Gegenwart einer Base die Esterfunktion verseift und in Stufe d) schliesslich in Gegenwart einer Mineralsäure zum Endprodukt decarboxyliert.

2. Verfahren nach Patentanspruch 1, dadurch gekennzeichnet, dass als Base für die Stufe a) ein Alkalialkoholat, ein Alkalihydroxid oder eine starke organische Base eingesetzt wird.

3. Verfahren nach mindestens einen der Patentansprüche 1 und 2, dadurch gekennzeichnet, daß die Stufe a) bei Temperaturen von 0 bis 180°C durchgeführt wird.

4. Verfahren nach mindestens einen der Patentansprüche 1 bis 3, dadurch gekennzeichnet, daß die Stufe a) in Gegenwart eines polaren Lösungsmittels, z.B. N,N'-Dimethylformamid durchgeführt wird.

5. Verfahren nach mindestens einen der Patentansprüche 1 bis 4, dadurch gekennzeichnet, daß der Ringschluß (Stufe b) bei Temperaturen zwischen 0 und 180°C in Gegenwart eines polaren Lösungsmittels erfolgt.

6. Verfahren nach mindestens einen der Patentansprüche 1 bis 5, dadurch gekennzeichnet, daß als Base für den Ringschluß ein Alkalialkoholat oder ein Alkalihydroxid eingesetzt wird.

7. Verfahren nach mindestens einen der Patentansprüche 1 bis 6, dadurch gekennzeichnet, daß als Base für Stufe c) eine wäßrige Lösung eines Alkalihydroxids eingesetzt wird.

8. Verfahren nach mindestens einen der Patentansprüche 1 bis 7, dadurch gekennzeichnet, daß die Verseifung (Stufe c) bei Temperaturen von 0 bis 100°C erfolgt.

9. Verfahren nach mindestens einen der Patentansprüche 1 bis 8, dadurch gekennzeichnet, daß die Decarboxylierung (Stufe d) mit einer wäßrigen Lösung einer Mineralsäure erfolgt.

10. Verfahren nach mindestens einen der Patentansprüche 1 bis 9, dadurch gekennzeichnet, daß Stufen a) und b) sowie die Stufen c) und d) ohne Isolierung des Zwischenproduktes durchgeführt werden.

11. 5,5-Bis-(alkoxycarbonyl)-3-alkoxy-2E-pentensäureester der Formel 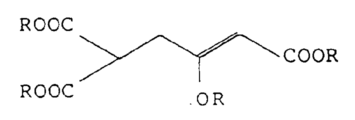 worin R gleich oder verschieden ist und Alkyl mit 1 bis 4 C-Atomen bedeutet, insbesondere
5,5-Bis-(ethoxycarbonyl)-3-methoxy-2E-pentensäuremethylester,
5,5-Bis-(methoxycarbonyl)-3-methoxy-2E-pentensäuremethylester,
5,5-Bis-(ethoxycarbonyl)-3-ethoxy-2E-pentensäureethylester.

## Claims

1. Process for the preparation of 1,3-cyclopentane dione, characterized in that in a first step a) a malonic acid ester is reacted in the presence of a base with a 4-halo-3-alkoxy-2E-butenic acid ester to a 5,5-bis-(alkoxycarbonyl)-3-alkoxy-2E-pentenic acid ester of the formula 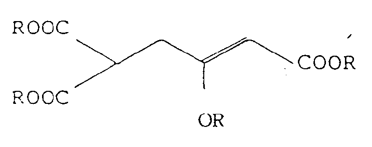 wherein the groups R each are the same or different and are alkyl groups having 1 to 4 carbon atoms, that in the second step b) the ring closure of the latter is effected in the presence of a base to obtain the corresponding salt of the 5-alkoxycarbonyl-3-alkoxy-2-cyclopentene-1-one of the formula 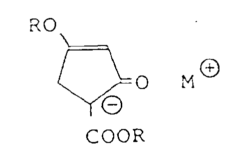 wherein M is sodium or potassium and R has the mentioned meaning, that in step c) the ester group is saponified in the presence of a base and that finally in step d) a decarboxylation is effected in the presence of a mineral acid to obtain the end product.

2. Process according to patent claim 1, characterized in that in step a) an alkali metal alcoholate, an alkali metal hydroxide or a strong organic base is used as base.

3. Process according to at least one of patent claims 1 and 2, characterized in that step a) is carried out at temperatures of from 0 to 180°C.

4. Process according to at least one of patent claims 1 to 3, characterized in that step a) is carried out in the presence of a polar solvent, such as N,N'-dimethyl formamide.

5. Process according to at least one of patent claims 1 to 4, characterized in that the ring closure (step b) is carried out in the presence of a polar solvent at temperatures between 0 and 180°C.

6. Process according to at least one of patent claims 1 to 5, characterized in that as base for the ring closure an alkali metal alcoholate or an alkali metal hydroxide is used.

7. Process according to at least one of patent claims 1 to 6, characterized in that in step c) an aqueous solution of an alkali metal hydroxide is used as base.

8. Process according to at least one of patent claims 1 to 7, characterized in that the saponification (step c) is carried out at temperatures of from 0 to 100 °C.

9. Process according to at least one of patent claims 1 to 8, characterized in that the decarboxylation (step d) is carried out with an aqueous solution of a mineral acid.

10. Process according to at least one of patent claims 1 to 9, characterized in that steps a) and b) as well as steps c) and d) are carried without isolation of the intermediate product.

11. 5,5-Bis-(alkoxycarbonyl)-3-alkoxy-2E-pentenic acid esters of the formula 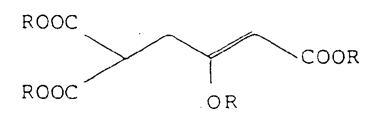 wherein R is the same or different and is alkyl having 1 to 4 carbon atoms, in particular
5,5-bis-(ethoxycarbonyl)-3-methoxy-2E-pentenic acid methyl ester,
5,5-bis-(methoxycarbonyl)-3-methoxy-2E-pentenic acid methyl ester,
5,5-bis-(ethoxycarbonyl)-3-ethoxy-2E-pentenic acid ethyl ester.

## Revendications

1. Procédé pour la préparation de 1,3-cyclopentanedione, caractérisé en ce que l'on met en réaction dans une première étape a) un ester de l'acide malonique en présence d'une base avec un ester de l'acide 4-halogen-3-alcoxy-2E-butène dans un ester de l'acide 5,5-bis-(alcoxy-carbonyl)-3-alcoxy-2E-pentène de la formule 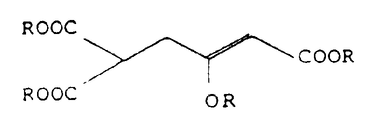 dans laquelle les restes R sont chaque fois identiques ou différents et signifient alkyle avec 1 à 4 atomes C, en ce que l'on procède à la cyclisation de celui-ci dans la deuxième étape b) en présence d'une base en le sel de 5-alcoxycarbonyl-3-alcoxy-2-cyclopentène-1-ones de la formule 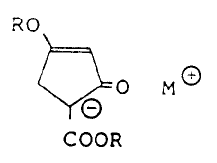 dans laquelle M signifie sodium ou potassium et R la signification indiquée, en ce qu'à l'étape c) en présence d'une base on saponifie la fonction ester et à l'étape d) finalement en présence d'un acide minéral on décarboxylise en produit final.

2. Procédé selon la revendication 1, caractérisé en ce qu'en tant que base pour l'étape a) on met en oeuvre un alcoolat alcalin, un hydroxyde alcalin ou une base organique forte.

3. Procédé selon au moins l'une des revendications 1 et 2, caractérisé en ce que l'étape a) est effectuée à des températures de 0 à 180°C.

4. Procédé selon au moins une des revendications 1 à 3, caractérisé en ce que l'étape a) est effectuée en présence d'un solvant polaire, par exemple N, N'-diméthylformamide.

5. Procédé selon au moins une des revendications 1 à 4, caractérisé en ce que la cyclisation (étape b) s'effectue à des températures entre 0 et 180°C en présence d'un solvant polaire.

6. Procédé selon au moins une des revendications 1 à 5, caractérisé en ce qu'en tant que base, on met en oeuvre pour la cyclisation un alcoolat alcalin ou un hydroxyde alcalin.

7. Procédé selon au moins une des revendications 1 à 6, caractérisé en ce qu'en tant que base, on met en oeuvre pour l'étape c) une solution aqueuse d'un hydroxyde alcalin.

8. Procédé selon au moins une des revendications 1 à 7, caractérisé en ce que la saponification (étape c) s'effectue à des températures de 0 à 100°C.

9. Procédé selon au moins une des revendications 1 à 8, caractérisé en ce que la décarboxylisation s'effectue (étape d) avec une solution aqueuse d'un acide minéral.

10. Procédé selon au moins une des revendications 1 à 9, caractérisé en ce que les étapes a) et b) ainsi que les étapes c) et d) s'effectuent sans isoler le produit intermédiaire.

11. Ester de l'acide 5,5-bis-(alcoxycarbonyl)-3-alcoxy-2E-pentène de la formule 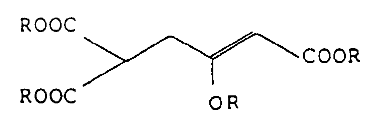 dans laquelle R est identique ou différent et signifie alkyle avec 1 à 4 atomes de carbone, notamment
l'ester de méthyle de l'acide 5,5-bis-(éthoxycarbonyl)-3-méthoxy-2E-pentène,
l'ester de méthyle de l'acide 5,5-bis-(méthoxycarbonyl)-3-méthoxy-2E-pentène,
l'ester d'éthyle de l'acide 5,5-bis-(éthoxycarbonyl)-3-éthoxy-2E-pentène.
